# EUROPEAN PATENT APPLICATION

(11) **EP 4 850 619 A1**
(43) Date of publication of application: **09.12.2026**
(21) Application number: 26182354.6
(22) Date of filing: 01.06.2026
(51) Int. Cl.: A61B 17/30

(54) **CHALAZION FORCEPS**

(30) Priority: 05.06.2025 GB 202508853; 07.08.2025 US 202519293203
(71) Applicant: Ojo, Oluropo Ebenezer, Sudbrooke, Lincoln LN2 2QU (GB)
(72) Inventor: Ojo, Oluropo Ebenezer, Sudbrooke, Lincoln LN2 2QU (GB)
(74) Representative: Loven, Keith James

(57) **Abstract**

Chalazion forceps comprise a pair of arms (1, 2) joined together at one end thereof, one arm (1) having at the other end a backing plate (5) and the other arm (2) having at the end thereof remote from the join a ring-like structure (6), the arms being resiliently deformable to bring the ring-like structure (6) into close alignment with the backing plate (5), and a ratchet mechanism (7) arranged between the two arms whereby to releasably latch the pair of arms into any of a plurality of positions relative to each other.

## Description

### FIELD OF THE INVENTION

This invention relates to chalazion forceps for use in the treatment by surgery of a chalazion.

### BACKGROUND OF THE INVENTION

The chalazion is the most common inflammatory lesion of the eyelid. A chalazion develops when lipid breakdown byproducts, potentially from bacterial enzymes or retained meibomian secretions, leak into the tarsal plate stroma, triggering a granulomatous inflammatory response (Alharbi, AS Int J Med Res Health Sci 2021, 10(9): 118-123). Histologically, this response is initially marked by neutrophils, followed by lymphocytes, plasma cells, macrophages, mononuclear cells, eosinophils, and multinucleated giant cells. The lesion is typically sterile. (Litoff D, Balin MW. Ocular infections and inflammation. Catalano RA, ed. Ocular Emergencies. WB Saunders; 1992. 468-471).

Chalazia typically present as a painless lump or swelling on either the upper or lower eyelid. They can occur unilaterally or bilaterally, and may involve single or multiple lesions.( Alharbi, 2021).

Despite a long history and the wealth of anecdotal evidence surrounding chalazion management, many literature have reported a lack of standardised protocol to the management of chalazion as there are many processes and procedures that have been introduced to manage or prevent chalazion (Kornhauser, Tom, et al., Management strategies for chalazia in pediatric patents: A scoping review, European Journal of Ophthalmology, 2025, and Park, James K. B.S.; Vyas, Charu B.A.; Dagi Glass, Lora R. M.D.. Chalazia: A Scoping Review to Identify the Evidence Behind Treatments. Ophthalmic Plastic and Reconstructive Surgery 41(2):p 134-142, March/April 2025. DOI: 10.1097/IOP. 0000000000002840). Kornhauser et al. emphasized the need for standardisation of chalazion management in paediatric age groups. However, surgical management is the most often studied in the literature as reported by Park et al in (2025) in their meta analysis study.

The first design of the chalazion forceps depicted in 1847 remains in use even now. However, the instrument has undergone several modifications since its initial design. These changes aim to improve both its application and safety for patients and Surgeons. (Maram Elsayed and Sultan al Kahtani: Chalazion Management: Evidence and Questions. Ophthalmic Pearls comprehensive. Spet 2015). The existing forceps design has a screw-threaded thumb-wheel engaged on a threaded post extending between the two arms of the forceps. While primarily used for chalazion extraction, this instrument also finds application in various other procedures, including: oral mucosal lesion procedures (biopsy, mucocele deroofing, and minor salivary gland biopsy), ear lobe repair, scrotal surgery, and nasal lesion removal on the ala nasi (Swetha B, et al, Chalazion Forceps - An Instrument Used for Mucocele and Biopsy of Oral Lesions a Literature Review, J Res Med Dent Sci, 2020, 8 (7): 138-141).

Thus, known chalazion forceps comprise a pair of arms joined together at one end thereof, one arm having at the other end a backing plate and the other arm having at the end thereof remote from the join a ring-like structure, the arms being resiliently deformable to bring the ring-like structure into close alignment with the backing plate.

The operation of the thumb-wheel to secure the forceps, and then to release the forceps after the operation is complete, requires care and dexterity and can cause discomfort for the patient.

### SUMMARY OF THE INVENTION

The chalazion forceps of the invention are characterised in that a sprung latching member extends from said one arm and through an aperture in the other arm, the latching member having a plurality of serrations along one side thereof and being biased to bring the serrations into engagement with one side of the aperture, the aperture being dimensioned to enable the latching member to be moved to bring the serrations out of engagement with the one side, releasing the pair of arms to move the backing plate and the ring-like structure away from each other.

The sprung latching member suitably extends through an aperture in the said one arm and has a portion extending therefrom and attached to the side of the said one arm.

The chalazion forceps may be formed of stainless steel or other medically acceptable metal or polymers permitting sterilisation.

In use, the ratchet mechanism locks the tip of the chalazion forceps in stages by clipping and allows the surgeon to achieve haemostasis without tissue crushing and minimizing the risk of pressure necrosis.

The chalazion forceps of the invention offers several advantages. It minimizes bleeding during surgery and provides a firm, immobile surface for incising lesions. The design facilitates a better secure grip on moist, slippery tissues, such as conjunctiva, oral mucosa. The clamp handle and size allows for easy manipulation of the surgical site, a significant benefit in procedures like chalazion extraction as well as in ear lobe repair where tissue fixation is challenging due to the small size and reduced surgical space.

The adjustable ratchet mechanism allows for easy and quick application of variable pressure, enabling haemostasis without tissue crushing and minimizing the risk of pressure necrosis. Finally, loosening the clamp by a single clip allows for precise identification and cauterization of bleeding points.

The latching member may be provided with a stop member projecting therefrom between the arms to limit the movement of the backing plate and ring-like structure towards each other, to maintain a minimum distance thereby to prevent inadvertent crushing of the eyelid between them.

Furthermore, the ratcheting mechanism facilitates a smoother and faster application and reapplication compare to the existing forceps. The current forceps with the screw mechanisms are cumbersome during application and reapplication and causes more patient discomfort whereas the new forceps ratcheting for holding and releasing tissue made the procedure smoother and less uncomfortable for the patients.

### BRIEF DESCRIPTION OF THE DRAWINGS

In the drawings:
Figure 1 is a plan view of conventional prior art chalazion forceps;
Figure 2 is a plan view of a forceps according to one embodiment of the invention;
Figure 3 is a view of the forceps of Figure 2 from above and to one side;
Figure 4 is an illustration showing the first stage in the use on an eyelid of the forceps shown in Figures 2 and 3; and
Figure 5 shows the second stage in the use, after inversion of the eyelid.

### DETAILED DESCRIPTION

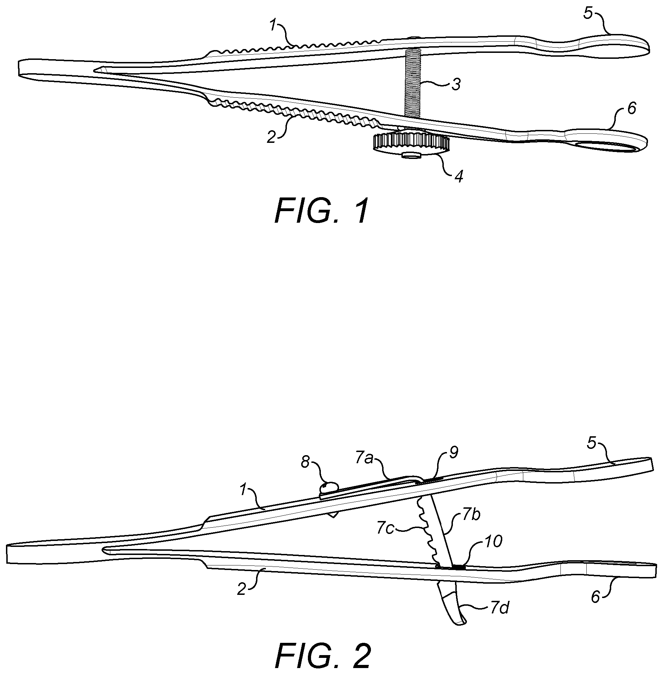

Referring first to Figure 1, conventional chalazion forceps for use in ophthalmologic surgery for chalazion extraction comprise a pair of arms 1 and 2, joined together at one end and resiliently deflectable towards each other. A screw-threaded member 3 extends from the first arm 1 and passes through an aperture in the other arm, where a thumb-wheel 4 engages the threaded member 3 and bears against the second arm 2 such that rotation of the thumb-wheel 4 in one direction draws the arms together against the resilience in the arms, or by rotation in the opposite direction allows the arms to move apart. The end of the first arm 1 is formed with a flat plate 5, while the end of the other arm 2 is formed with a ring 6 which corresponds to the plate 5 and can be brought into close parallel proximity with the plate 5 when the arms are drawn together.

In use, the chalazion forceps is positioned with the ring beneath the eyelid and around the chalazion and the flat plate is then pressed against the external surface of the eyelid to achieve haemostasis. This is done by rotating the thumb-wheel 4 while holding the forceps in place. The eyelid can then be inverted to permit the surgery for chalazion extraction to be carried out.

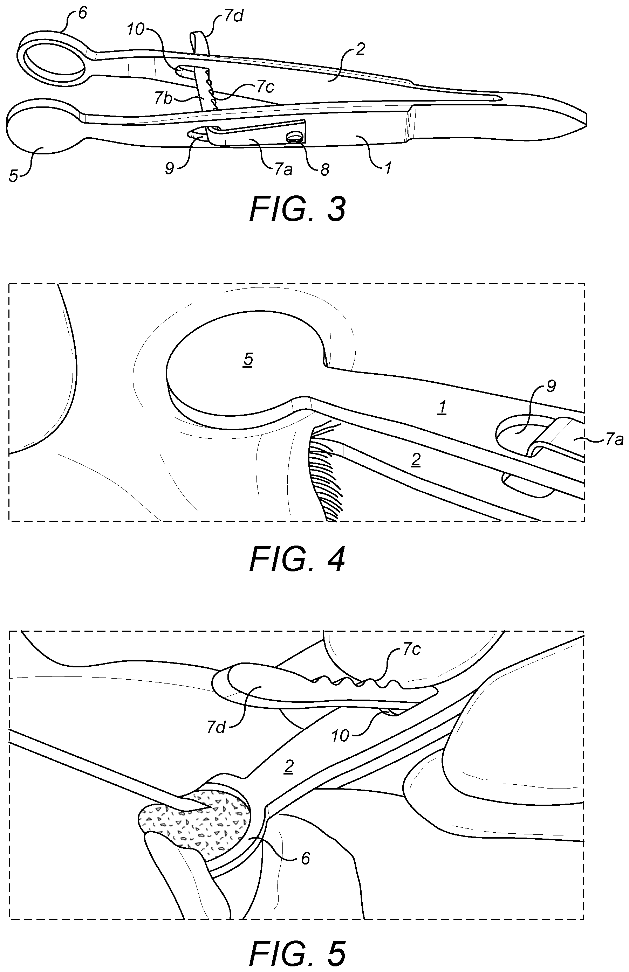

Referring now to Figures 2 and 3, in which like components to those of the forceps shown in Figure 1 have the same reference numerals, an L-shaped ratchet member 7 has a first leg 7a attached at one end thereof to the outer surface of the first arm 1 by a rivet or screw 8 (a weld could alternatively be used), while the second leg 7b, which is provided with a series of serrations 7c along one side, passes through an elongate slot 9 in the arm 1 and then through an elongate slot 10 in the second arm 2. The first leg 7a of the ratchet member 7 is formed to be resiliently-deformable and the attachment at one end thereof enables it to act as a spring configured to bias the serrations 7c on the second leg 7b against the end of the slot 10 to provide the ratchet action. As the two arms 1 and 2 are squeezed together, successive serrations or slots 7c engage the end of the slot 10 to hold the arms 1 and 2 in positions successively closer together. The surgeon can thus press the arms together to apply the desired pressure between the plate 5 and the ring 6 and the ratchet will hold the position until the arms are released by pressure on the free end 7d of the second leg 7b where it projects from the second arm 2. This can be done with one finger or the thumb. The second leg 7b can be configured with a stop to prevent the accidental application of a crushing pressure to the eyelid, in use.

Figures 4 and 5 illustrate the use of the forceps in the removal of a chalazion. In Figure 4, the forceps has been positioned with the ring 6 beneath the eyelid 11 and surrounding the chalazion, with the flat plate 5 pressed against the outer surface of the eyelid, with the arms 1 and 2 of the forceps squeezed together and held in position by the ratchet. The forceps is then used to lift and invert the eyelid 11 to expose the chalazion 12 for surgical attention.

While the invention has been described in relation to the treatment of chalazia, it will be appreciated that the forceps will be useful in connection with other procedures, including veterinary procedures, and the size of the forceps can be adapted to suit the particular procedures. In some procedures, it may be advantageous to provide the plate 5 with an aperture therethrough, and while the plate is referred to as flat, it may be desirable for use with some procedures to provide the backing plate with a dished or other non-flat shape.

## Claims

1. Chalazion forceps, comprising a pair of arms (1, 2) joined together at one end thereof, one arm (1) having at the other end a backing plate (5) and the other arm having at the end thereof remote from the join a ring-like structure (6), the arms (1, 2) being resiliently deformable to bring the ring-like structure (6) into close alignment with the backing plate (5), **characterised in that** a sprung latching member (7) extends from said one arm (1) and through an aperture (10) in the other arm (2), the latching member (7) having a plurality of serrations (7c) along one side thereof and being biased to bring the serrations into engagement with one side of the aperture, the aperture (10) being dimensioned to enable the latching member (7) to be moved to bring the serrations (7c) out of engagement with the one side, releasing the pair of arms (1,2) to move the backing plate (5) and the ring-like structure (6) away from each other.

2. Chalazion forceps according to Claim 1, wherein the sprung latching member (7) extends through an aperture (9) in the said one arm (1) and has a portion (7a) extending therefrom and attached to the side of the said one arm.

3. Chalazion forceps according to Claim 1 or 2, wherein the backing plate has an aperture therethrough.

4. Chalazion forceps according to Claim 1, 2 or 3, wherein the latching member is provided with a stop member projecting therefrom between the arms to limit the movement of the backing plate and ring-like structure towards each other, to maintain a minimum distance thereby to prevent inadvertent crushing of the eyelid between them.
